# EUROPEAN PATENT SPECIFICATION

(11) **EP 1 194 033 B1**
(45) Date of publication and mention of the grant of the patent: **19.12.2007**
(21) Application number: 00932348.6
(22) Date of filing: 12.05.2000
(51) Int. Cl.: A01K 67/00, C12N 5/00, C07K 14/81

(54) **TRANSGENICALLY PRODUCED ANTITHROMBIN III AND MUTANTS THEREOF**
TRANSGENE HERGESTELLTE ANTITHROMBIN III UND DEREN MUTANTEN
ANTITHROMBINE III PRODUITE DE MANIERE TRANSGENIQUE ET MUTANTS DE CELLE-CI

(30) Priority: 13.05.1999 US 134174 P
(43) Date of publication of application: 10.04.2002
(73) Proprietor: GTC Biotherapeutics, Inc., Framingham, MA 01702 (US)
(72) Inventor: MEADE, Harry, Newton, MA 02158 (US); BOURDON, Paul, R., Southborough, MA (US)
(74) Representative: Ruffles, Graham Keith
(86) International application number: PCT/US2000/013052
(87) International publication number: WO 2000/069256

(56) References cited:
- EP-A- 0 384 122
- WO-A-00/20026
- WO-A-99/58098
- WO-A1-95/03409
- WO-A1-96/10638
- WO-A1-96/26268
- US-A- 5 756 687
- US-A- 5 843 705
- BEAUCHAMP N J ET AL: "Antithrombins wibble and wobble (T85M/K): Archetypal conformational diseases with in vivo latent-transition, thrombosis, and heparin activation" BLOOD, vol. 92, no. 8, 15 October 1998 (1998-10-15), pages 2696-2706, XP002279339 ISSN: 0006-4971
- PERRY J ET AL: "Two novel antithrombin variants, Asn187Asp and Asn187Lys, indicates a functional role for asparagine 187" BLOOD COAGULATION AND FIBRINOLYSIS, vol. 6, no. 1, 1995, pages 51-54, XP009030550 ISSN: 0957-5235
- BRUCE DAVID ET AL: "Thromboembolic Disease Due to Thermolabile Conformational Changes of antithrombin Rouen-VI (187 Asn fwdarw ASP)" JOURNAL OF CLINICAL INVESTIGATION, vol. 94, no. 6, 1994, pages 2265-2274, XP009030551 ISSN: 0021-9738
- KRIDEL S J ET AL: "Lysine residue 114 in human antithrombin III is required for heparin pentasaccharide-mediated activation" JOURNAL OF BIOLOGICAL CHEMISTRY 1997 UNITED STATES, vol. 272, no. 12, 1997, pages 7656-7660, XP002279340 ISSN: 0021-9258
- SAKURAGAWA N ET AL: "ABNORMAL ANTITHROMBIN III: ABNORMALITIES OF HEPARIN OR PROTEASE BINDING DOMAIN" RINSHO BYORI / JAPANESE JOURNAL OF CLINICAL PATHOLOGY, NIPPON RINSHO BYORI GAKKAI, TOKYO, JP, vol. 41, no. 5, May 1993 (1993-05), pages 492-505, XP009021690 ISSN: 0047-1860
- KRIDEL S J ET AL: "Requirement of lysine residues outside of the proposed pentasaccharide binding region for high affinity heparin binding and activation of human antithrombin III" JOURNAL OF BIOLOGICAL CHEMISTRY 1996 UNITED STATES, vol. 271, no. 34, 1996, pages 20935-20941, XP002279341 ISSN: 0021-9258
- OLSON S T ET AL: "Effect of individual carbohydrate chains of recombinant antithrombin on heparin affinity and on the generation of glycoforms differing in heparin affinity." ARCHIVES OF BIOCHEMISTRY AND BIOPHYSICS. UNITED STATES 15 MAY 1997, vol. 341, no. 2, 15 May 1997 (1997-05-15), pages 212-221, XP002279342 ISSN: 0003-9861
- BJÖRK I ET AL: "DECREASED AFFINITY OF RECOMBINANT ANTITHROMBIN FOR HEPARIN DUE TO INCREASED GLYCOSYLATION" BIOCHEMICAL JOURNAL, PORTLAND PRESS, LONDON, GB, vol. 286, 1992, pages 793-800, XP000933704 ISSN: 0264-6021
- WARDELL M R ET AL: "PREPARATIVE INDUCTION AND CHARACTERIZATION OF L-ANTITHROMBIN: A STRUCTURAL HOMOLOGUE OF LATENT PLASMINOGEN ACTIVATOR INHIBITOR-1" BIOCHEMISTRY, AMERICAN CHEMICAL SOCIETY. EASTON, PA, US, vol. 36, no. 42, 1997, pages 13133-13142, XP002909962 ISSN: 0006-2960
- CARRELL R W ET AL: "BIOLOGICAL IMPLICATIONS OF A 3 AA STRUCTURE OF DIMERIC ANTITHROMBIN" STRUCTURE, CURRENT BIOLOGY LTD., PHILADELPHIA, PA, US, vol. 2, no. 4, 15 April 1994 (1994-04-15), pages 257-270, XP002950393 ISSN: 0969-2126
- EDMUNDS T. ET. AL.: 'Transgenically produced human antithrombin: structural and functional comparision of human plasma-derived antithrombin' BLOOD vol. 91, no. 12, 15 June 1998, pages 4561 - 4571, XP000892719
- O'REILLY ET. AL.: 'Antiangiogenic activity of the cleaved conformation of the seprin antithrombin' SCIENCE vol. 285, 17 September 1999, pages 1926 - 1928, XP002135982

## Description

### Background of the Invention

A growing number of recombinant proteins are being developed for therapeutic and diagnostic applications; however, many of these proteins may be difficult or expensive to produce in a functional form in the required quantities using conventional methods. Conventional methods involve inserting the gene responsible for the production of a particular protein into host cells such as bacteria, yeast, or mammalian cells, and then growing the cells in culture media. The cultured cells then synthesize the desired protein. Traditional bacteria or yeast systems may be unable to produce many complex proteins in a functional form. While mammalian cells can reproduce complex proteins, they are generally difficult and expensive to grow, and produce only mg/L quantities of protein.

The application of transgenic technology to the commercial production of recombinant proteins in the milk of transgenic animals offers significant advantages over traditional methods of protein production. These advantages include a reduction in the total amount of required capital expenditures, elimination of the need for capital commitment to build facilities early in the product development life cycle, and lower direct production cost per unit for complex proteins. Of key importance is the likelihood that, for certain complex proteins, transgenic production may represent the only technologically and economically feasible method of commercial production.

In its active state, antithrombin III (ATIII) is a serine protease inhibitor which inhibits thrombin and the activated forms of factors X, VII, IX, XI, and XII. It is normally present in serum at levels of 14-20 mg/L. Decreased levels of ATIII may be found in the serum of individuals who have either a hereditary deficiency of ATIII or an acquired deficiency, which can result from a number of pathologic conditions. The conventional treatment for hereditary ATIII deficiency is protein replacement therapy, which may also be effective in treating some acquired deficiencies.

Current methods of obtaining ATIII involves isolating the protease inhibitor from blood plasma. However, the use of plasm-based ATIII presents various problems due to the many components in plasma, including: variation between lots; immunogenicity problems; and biohazardous risks due to viral contamination.

WO 96/26268 describes the production of wild-type human Antithrombin III in the milk of transgenic animals. Edmund T et al, Blood Vol. 91 no. 12 pages 4561 to 4571 describes the structural and functional analysis of transgenically produced human Antithrombin.

Various forms of mutant Antithrombin III are described in the literature, including Beauchamp N J et al, Blood, Vol. 92, no. 8, pages 2696 to 2708, which describes a T85M/K mutation; Perry J et al, Blood, Coagulation and Fibrinolysis, vol. 6, no. 1, 1995 pages 51 to 54, which describes Asn187Asp or Asn187Lys mutations; Bruce D et al, Journal of Clinical Investigation, vol. 94, no. 6, pages 2265-2274, which describes the consequences of the Asn187Asp mutation; and Sakuragawa N et al, Japanese Journal of Clinical Pathology, vol. 41, no. 5, pages 492 to 505, which describes C-terminal deletion mutants of Antithrombin III. However, all of these publications describe naturally occurring mutations, and do not suggest that such mutants may be useful as medicaments.

### Summary of Invention

The invention is based, in part, on the discovery that mutant forms of Antithrombin III (ATIII) can be transgenically produced. In its active state ATIII is a serine protease inhibitor that inhibits thrombin as well as other serine proteases of the cascade. After changing its confirmation from the stressed or active state to a latent or cleaved state, ATIII can also inhibit angiogenesis. It was discovered that various mutants which facilitate the anticoagulant and/or antiangiogenic activity of ATIII can be transgenically produced. Thus, the invention generally relates to transgenically produced human Antithrombin III (tgATIII).

Specifically, the invention relates to transgenically produced mutated human Antithrombin III (tgATIIIₘᵤₜ) having an antiangiogenic activity, wherein the reactive loop of ATIII is unreactive, for use as a medicament. Other embodiments relate to methods of producing such transgenic ATIII in the milk of a transgenic animal. The mutant Antithrombin III has an unreactive reactive loop, for example, the reactive loop is folded (e.g., the reactive loop is folded into the beta sheet) or the reactive loop is partially or wholly removed.

In a preferred embodiment, the ATIII is mutated by altering at least one alpha helix underlying the beta sheet, e.g., by insertion, deletion, or a substitution of at least one nucleotide of the sequence encoding an alpha helix of ATIII, e.g., human ATIII. The alpha helix can be mutated by, for example, one or more of changing threonine at position 85 to methionine; changing threonine at position 85 to lysine; and changing asparagine at position 187 to an aspartic acid. In a preferred embodiment, the mutation alters the reactive loop, e.g., by destabilizing the beta sheet by allowing the beta strand of the reactive loop to fold down in between the third/fourth and fifth/sixth strands of the beta sheet. In a preferred embodiment, an altered alpha helix tg ATIIIₘᵤₜ forms the latent form of ATIII more readily than wild type ATIII, e.g., by spontaneous conversion and/or at lower temperatures.

In a preferred embodiment, the ATIII is mutated by removing a portion or all of the C-terminus of ATIII. In a preferred embodiment, the C-terminus is removed at about 70, 60, 50 amino acids or less from the C-terminus. Preferably, the C-terminus is removed at the elastase site (e.g., about 43 amino acids from the C-terminus); or, the thrombin cleavage site (e.g., about 39 amino acids from the C-terminus). In a preferred embodiment, the C-terminus mutated ATIII alter the reactive loop, e.g., by allowing the beta strand of the reactive loop to fold into the beta sheet. In a preferred embodiment, C-terminus mutated ATIII is secreted from a transgenic animal in an active form.

ATIII may be mutated by removing at least one, two, three, four glycosylation site(s), The term "deglycosylated" as used herein refers to an ATIII mutant in which at least one, some or all of the glycosylation sites are removed. The deglycosylated ATIII mutant may comprise an asparagine at position 135 which is followed by a serine at the third position. In another embodiment, the asparagine at position 135 is changed to a glutamine. In an embodiment, two, three, four of the glycosylation sites can be removed, e.g., the asparagine is replaced with a glutamine. In one embodiment, the deglycosylated ATIII mutant has one or more of the following activities: it interacts, e.g., binds heparin tigthter than wild type ATIII; it interacts with, e.g., binds, heparin sulfates on endothelial cell surface; inhibits or reduces procoagulant activity; increases the half-life of the mutated ATIII, e.g., as compared to wildtype ATIII.

In one embodiment, ATIII is mutated by decreasing heparin binding, e.g., by mutating one or more amino acid outside of the pentasaccharide-binding domain, e.g., by insertion, deletion or substitution. In one embodiment, ATII is mutated by substituting one or more amino acid outside of the pentasaccharide-binding domain, e.g., by changing lysine at position 114 to a glutamine. In one embodiment, the heparin binding mutated form of ATIII substantially reduces heparin binding. In one embodiment, the heparin binding mutant of ATIII has inhibitory activity comparable to wild type ATIII inhibitory activity but is not accelerated in the presence of heparin.

The transgenically produced tgATIII ₘᵤₜ may be produced in a transgenic organism, i.e., a transgenic plant or animal. Preferred transgenic animals include: mammals; birds; reptiles; and amphibians. Suitable mammals include: ruminants; ungulates; domesticated mammals; and dairy animals. Particularly preferred animals include: goats, sheep, camels, cows, pigs, horses, oxen, rabbits and Ilamas. Suitable birds include chickens, geese, and turkeys. Where the transgenic protein is secreted into the milk of a transgenic animal, the animal should be able to produce at least 1, and more preferably at least 10, or 100, liters of milk per year.

In preferred embodiments, the transgenically produced tgATIII ₘᵤₜ is made in a mammary gland of the transgenic mammal, e.g., a ruminant, e.g., a goat.

In preferred embodiments, the transgenically produced tgATIII ₘᵤₜ is secreted into the milk of the transgenic mammal, e.g., a ruminant, e.g., a goat.

In preferred embodiments, the transgenically produced tgATIII ₘᵤₜ is made under the control of a mammary gland specific promoter, e.g., a milk specific promoter, e.g., a milk serum protein or casein promoter. The milk specific promoter can is a casein promoter, beta lactoglobulin promoter, whey acid protein promoter, or lactalbumin promoter.

In some embodiments, the tgATIII ₘᵤₜ is made under the control of a bladder, or egg specific promoter and tgATIIIₘᵤₜ is secreted into the urine or into an egg.

The tgATIII ₘᵤₜ is a mutation of the nucleotide and/or amino acid sequence of human, ATIII.

In preferred embodiments, the preparation includes at least 0.1,1, 10, or 100 milligrams of tgATIII ₘᵤₜ. In preferred embodiments, the preparation includes at least 0.1, 1, 10, or 100 grams of tgATIIIₘᵤₜ.

In preferred embodiments, the preparation includes at least 1, 10, 100, or 500 milligrams per milliliter of tgATIIIₘᵤₜ.

We also describe an isolated nucleic acid molecule including a tgATIII ₘᵤₜ protein-coding sequence operatively linked to a tissue specific promoter, e.g., a mammary gland specific promoter sequence that results in the secretion of the protein in the milk of a transgenic mammal. The tgATIII ₘᵤₜ can be, e.g., a tgATIII ₘᵤₜ described herein.

In preferred embodiments, the promoter is a milk specific promoter, e.g., a milk serum protein or casein promoter. The milk specific promoter can is a casein promoter, beta lactoglobulin promoter, whey acid protein promoter, or lactalbumin promoter.

In preferred embodiments, the promoter is a bladder, or egg specific promoter and tgATIIIₘᵤₜ is secreted into the urine or into an egg.

We also describe a method of making transgenic tgATIII ₘᵤₜ or a preparation of transgenic tgATIII ₘᵤₜ described herein. The method includes:
providing a transgenic organism, i.e., a transgenic animal or plant, which includes a transgene which directs the expression of tgATIII ₘᵤₜ;
allowing the transgene to be expressed; and recovering transgenically produced tgATIII ₘᵤₜ or a preparation of transgenically produced tgATIII ₘᵤₜ, from the organism or from a product produced by the organism, e.g., milk, seeds, hair, blood, eggs, or urine.

In preferred embodiments, the method further includes:
inserting a nucleic acid which directs the expression of tgATIII ₘᵤₜ into a cell and allowing the cell to give rise to a transgenic organism;

The tgATIII ₘᵤₜ can be, e.g., any tgATIII ₘᵤₜ described herein.

Preferred transgenic animals include; mammals; birds; reptiles; and amphibians. Suitable mammals include: ruminants; ungulates; domesticated mammals; and dairy animals. Particularly preferred animals include : goats, sheep, camels, cows, pigs, horses, oxen, rabbits and Ilamas. Suitable birds include chickens, gesse, and turkeys. Where the transgenic protein is secreted into the milk of a transgenic animal, the animal should be able to produce at least 1, and more preferably at least 10, or 100, liters of milk per year.

In preferred embodiments, the transgenically produced tgATIIIₘᵤₜ is made in a mammary gland of the transgenic mammal, e.g., a ruminant, e.g., a goat.

In preferred embodiments, the transgenically produced tgATIIIₘᵤₜ is secreted into the milk of the transgenic mammal, e.g., a ruminant, e.g., a goat

In preferred embodiments, the transgenically produced tgATIII ₘᵤₜ is made under the control of a mammary gland specific promoter, e.g., a milk specific promoter, e.g., a milk serum protein or casein promoter. The milk specific promoter can is a casein promoter, beta lactoglobulin promoter, whey acid protein promoter, or lactalbumin promoter.

In preferred embodiments, the tgATIII ₘᵤₜ is made under the control of a bladder, or egg specific promoter and tgATIII ₘᵤₜ is secreted into the urine or into an egg.

In preferred embodiments, the tgATIII ₘᵤₜ is a mutation of the nucleotide and/or amino acid sequence of mammalian or primate, preferably human, ATIII.

In preferred embodiments, the preparation includes at least 1, 10, or 100 milligrams of tgAIII ₘᵤₜ. In preferred embodiments, the preparation includes at least 1,10, or 100 grams of tgATIII ₘᵤₜ.

In preferred embodiments, the preparation includes at least 1, 10, 100, or 500 milligrams per milliliter of tgATIII ₘᵤₜ.

We also describe a method for providing a transgenic preparation which includes tgATIII ₘᵤₜ in the milk of a transgenic mammal including :
obtaining milk from a transgenic mammal having introduced into its germline a tgATIII ₘᵤₜ protein-coding sequence operatively linked to a promoter sequence that result in the expression of the protein-coding sequence in mammary gland epithelial cells, thereby secreting the tgATIII ₘᵤₜ in the milk of the mammal to provide the preparation.

The tgATIII ₘᵤₜ can be, e.g., any tgATIII ₘᵤₜ described herein.

Suitable mammals include: ruminants; ungulates; domesticated mammals; and dairy animals. Particularly preferred mammals include: goats, sheep, camels, cows, pigs, horses, oxen, and llamas. The transgenic mammal should be able to produce at least 0.1, 1, and more preferably at least 10, or 100, liters of milk per year.

In preferred embodiments, the transgenically produced tgATIII ₘᵤₜ is made in a mammary gland of the transgenic mammal, e.g., a ruminant, e.g., a goat

In preferred embodiments, the transgenically produced tgATIIIₘᵤₜ is secreted into the milk of the transgenic mammal, e.g., a ruminant, e.g., a goat

In preferred embodiments, the transgenically produced tgATIII ₘᵤₜ is made under the control of a mammary gland specific promoter, e.g., a milk specific promoter, e.g., a milk serum protein or casein promoter. The milk specific promoter can is a casein promoter, beta lactoglobulin promoter, whey acid protein promoter, or lactalbumin promoter.

In preferred embodiments, the milk includes at least 1, 10, 100, 500, 1,000, or 2,000 milligrams per milliliter, of tgATIII ₘᵤₜ.

We also describe a transgenic organism, which expresses a transgenic tgATIII ₘᵤₜ, preferably tgATIIIₘᵤₜ derived from human ATIII, and from which a transgenic preparation of tgATIII ₘᵤₜ can be obtained.

The tgATIIIₘᵤₜ can be, e.g., any tgATIII ₘᵤₜ described herein.

The transgenic organism is a transgenic plant or animal. Preferred transgenic animals include: mammals; birds; reptiles; and amphibians. Suitable mammals include: ruminants; ungulates; domesticated mammals; and dairy animals. Particularly preferred animals include: goats, sheep, camels, cows, pigs, horses, oxen, rabbits and Ilamas. Suitable birds include chickens, geese, and turkeys. Where the transgenic protein is secreted into the milk of a transgenic animal, the animal should be able to produce at least 0.1, 1, and more preferably at least 10, or 100, liters of milk per year.

In preferred embodiments, the transgenically produced tgATIIIₘᵤₜ is made in a mammary gland of the transgenic mammal, e.g., a ruminant, e.g., a goat.

In preferred embodiments, the transgenically produced tgATIII ₘᵤₜ is secreted into the milk of the transgenic mammal, e.g., a ruminant, e.g., a goat.

In preferred embodiments, the transgenically produced tgATIIIₘᵤₜ is made under the control of a mammary gland specific promoter, e.g., a milk specific promoter, e.g., a milk serum protein or casein promoter. The milk specific promoter can is a casein promoter, beta lactoglobulin promoter, whey acid protein promoter, or lactalbumin promoter.

In preferred embodiments, the tgATIII ₘᵤₜ is made under the control of a bladder, or egg specific promoter and is tgATIIIₘᵤₜ secreted into the urine or into an egg.

In preferred embodiments, the tgATIII ₘᵤₜ is a mutated nucleotide or amino acid sequence from mammalian or primate, preferably human, ATIII.

In preferred embodiments, the preparation includes at least 1, 10, or 100 milligrams of tgATIII ₘᵤₜ. In preferred embodiments, the preparation includes at least 1, 10, or 100 grams of tgATIII ₘᵤₜ

In preferred embodiments, the preparation includes at least 1,10, 100, or 500 milligrams per milliliter of tgATIII ₘᵤₜ.

We also describe a pharmaceutical composition including a therapeutically effective amount of transgenic tgATIII ₘᵤₜ, or a transgenic preparation of tgATIIIₘᵤₜ, e.g., a tgATIIIₘᵤₜ described herein, and a pharmaceutically acceptable carrier.

The transgenic tgATIIIₘᵤₜ or tgATIIIₘᵤₜ preparation can be made, e.g., by any method or organism described herein.

A preparation, as used herein, refers to a plurality of molecules produced by one or more transgenic animals. In one aspect, it can include molecules of differing glycosylation or it can be homogenous in this regard.

A purified preparation, substantially pure preparation of a polypeptide, or an isolated polypeptide as used herein, means, in the case of a transgenically produced polypeptide, a polypeptide that has been separated from at least one other protein, lipid, or nucleic acid with which it occurs in the transgenic animal or in a fluid, e.g., milk, or other substance, e.g., an egg, produced by the transgenic animal. The polypeptide is preferably separated from substances, e.g., antibodies or gel matrix, e.g., polyacrylamide, which are used to purify it. The polypeptide is preferably constitutes at least 10, 20, 50 70, 80 or 95% dry weight of the purified preparation. Preferably, the preparation contains: sufficient polypeptide to allow protein sequencing; at least 1, 10, or 100 µg of the polypeptide; at least 1, 10, or 100 mg of the polypeptide.

A substantially pure nucleic acid, is a nucleic acid which is one or both of: not immediately contiguous with either one or both of the sequences, e.g., coding sequences, with which it is immediately contiguous (i.e., one at the 5' end and one at the 3' end) in the naturally-occurring genome of the organism from which the nucleic acid is derived; or which is substantially free of a nucleic acid sequence with which it occurs in the organism from which the nucleic acid is derived. The term includes, for example, a recombinant DNA which is incorporated into a vector, e.g., into an autonomously replicating plasmid or virus, or into the genomic DNA of a prokaryote or eukaryote, or which exists as a separate molecule (e.g., a cDNA or a genomic DNA fragment produced by PCR or restriction endonuclease treatment) independent of other DNA sequences. Substantially pure DNA also includes a recombinant DNA which is part of a hybrid gene encoding additional tgATIIIₘᵤₜ sequence.

The terms peptides, proteins, and polypeptides are used interchangeably herein.

As used herein, the term "transgenic sequence" refers to a nucleic acid sequence (e.g., encoding one or more human proteins), which is inserted by artifice into a cell. The transgenic sequence, also referred to herein as a transgene, can become part of the genome of an animal which develops in whole or in part from that cell. In embodiments of the invention, the transgenic sequence is integrated into the chromosomal genome. If the transgenic sequence is integrated into the genome it results, merely by virtue of its insertion, in a change in the nucleic acid sequence of the genome into which it is inserted. A transgenic sequence can be partly or entirely species-heterologous, i.e., the transgenic sequence, or a portion thereof, can be from a species which is different from the cell into which it is introduced. A transgenic sequence can be partly or entirely species-homologous, i.e., the transgenic sequence, or a portion thereof, can be from the same species as is the cell into which it is introduced. If a transgenic sequence is homologous (in the sequence sense or in the species-homologous sense) to an endogenous gene of the cell into which it is introduced, then the transgenic sequence, preferably, has one or more of the following characteristics: it is designed for insertion, or is inserted, into the cell's genome in such a way as to alter the sequence of the genome of the cell into which it is inserted (e.g., it is inserted at a location which differs from that of the endogenous gene or its insertion results in a change in the sequence of the endogenous endogenous gene); it includes a mutation, e.g., a mutation which results in misexpression of the transgenic sequence; by virtue of its insertion, it can result in misexpression of the gene into which it is inserted, e.g., the insertion can result in a knockout of the gene into which it is inserted. A transgenic sequence can include one or more transcriptional regulatory sequences and any other nucleic acid sequences, such as introns, that may be necessary for a desired level or pattern of expression of a selected nucleic acid, all operably linked to the selected nucleic acid. The transgenic sequence can include an enhancer sequence and or sequences which allow for secretion.

The term "heterologous promoter" as used herein, refers to a promoter which is not normally associated with the gene it controls or which is heterologous to the mammal into which it is introduced.

Mammals are defined herein as all animals, excluding humans, that have mammary glands and produce milk.

As used herein, a "dairy animal" refers to a milk producing animal. In preferred embodiments, the dairy animal produce large volumes of milk and have long lactating periods, e.g., cows or goats.

As used herein, the term "plant" refers to either a whole plant, a plant part, a plant cell, or a group of plant cells. The class of plants which can be used in the method of the invention is generally as broad as the class of higher plants amenable to transformation techniques, including both monocotyledonous and dicotyledonous plants. It includes plants of a variety of ploidy levels, including polyploid, diploid and haploid.

The term "pharmaceutically acceptable composition" refers to compositions which comprise a therapeutically-effective amount of tgATIII ₘᵤₜ, formulated together with one or more pharmaceutically acceptable carrier(s).

Other features and advantages of the invention will be apparent from the following detailed description, and from the claims.

### Brief Description of the Drawings

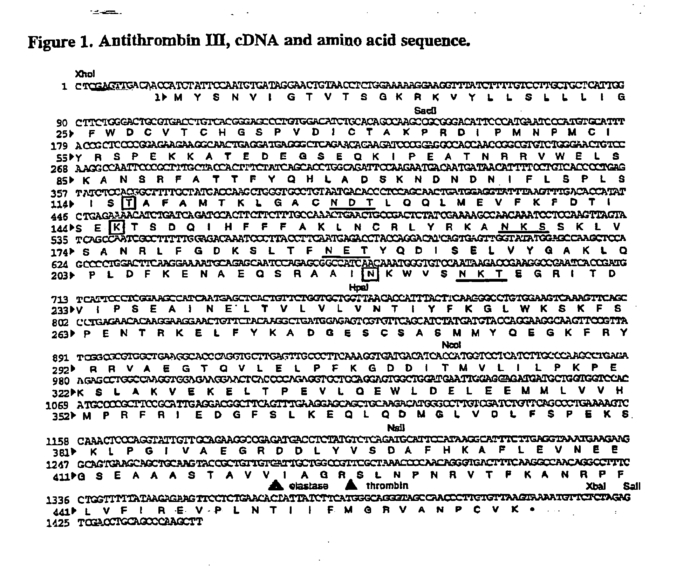

Figure 1 depicts human ATIII cDNA and amino acid sequences.

### Detailed Description of the Invention

This invention is based upon the discovery that mutated forms of ATIII can facilitate anticoagulant and/or antiangiogenic activity of ATIII. Such mutated forms of ATIII can be transgenically produced and secreted into the milk of a transgenic animal.

### Transgenic Mammals

Methods for generating non-human transgenic mammals which can be used as a source of somatic cells in the invention are known in the art. Such methods can involve introducing DNA constructs into the germ line of a mammal to make a transgenic mammal. For example, one or several copies of the construct may be incorporated into the genome of a mammalian embryo by standard transgenic techniques. In addition, non-human transgenic mammals can be produced using a somatic cell as a donor cell. The genome of the somatic cell can then be inserted into an oocyte and the oocyte can be fused and activated to form a reconstructed embryo. For example, methods of producing transgenic animals using a somatic cell are described in PCT Publication WO 97/07669.

Although goats are a preferred source of genetically engineered somatic cells, other non-human mammals can be used. Preferred non-human mammals are ruminants, e.g., cows, sheep, camels or goats. Goats of Swiss origin, e.g., the Alpine, Saanen and Toggenburg breed goats, are useful in the methods described herein. Additional examples of preferred non-human animals include oxen, horses, llamas, and pigs. The mammal used as the source of genetically engineered cells will depend on the transgenic mammal to be obtained by the methods of the invention as, by way of example, a goat genome should be introduced into a goat functionally enucleated oocyte.

Preferably, the somatic cells for use in the invention are obtained from a transgenic goat. Methods of producing transgenic goats are known in the art. For example, a transgene can be introduced into the germline of a goat by microinjection as described, for example, in Ebert et al. (1994) Bio/Technology 12:699, hereby incorporated by reference.

Other transgenic non-human animals to be used as a source of genetically engineered somatic cells can be produced by introducing a transgene into the germline of the non-human animal. Embryonal target cells at various developmental stages can be used to introduce transgenes. Different methods are used depending on the stage of development of the embryonal target cell. The specific line(s) of any animal used to practice this invention are selected for general good health, good embryo yields, good pronuclear visibility in the embryo, and good reproductive fitness. In addition, the haplotype is a significant factor.

### Transfected Cell Lines

Genetically engineered cell lines can be used to produce a transgenic animal. A genetically engineered construct can be introduced into a cell via conventional transformation or transfection techniques. As used herein, the terms "transfection" and "transformation" include a variety of techniques for introducing a transgenic sequence into a host cell, including calcium phosphate or calcium chloride co-precipitation, DEAE-dextrane-mediated transfection, lipofection, or electroporation. In addition, biological vectors, e.g., viral vectors can be used as described below. Suitable methods for transforming or transfecting host cells can be found in Sambrook et al., Molecular Cloning: A Laboratory Manuel, 2nd ed., Cold Spring Harbor Laboratory, (Cold Spring Harbor Laboratory Press, Cold Spring Harbor, NY, 1989), and other suitable laboratory manuals.

Two useful approaches are electroporation and lipofection. Brief examples of each are described below.

The DNA construct can be stably introduced into a donor somatic cell line by electroporation using the following protocol: somatic cells, e.g., fibroblasts, e.g., embryonic fibroblasts, are resuspended in PBS at about 4 x 10⁶ cells/ml. Fifty micorgrams of linearized DNA is added to the 0.5 ml cell suspension, and the suspension is placed in a 0.4 cm electrode gap cuvette (Biorad). Electroporation is performed using a Biorad Gene Pulser electroporator with a 330 volt pulse at 25 mA, 1000 microFarad and infinite resistance. If the DNA construct contains a Neomyocin resistance gene for selection, neomyocin resistant clones are selected following incubation with 350 microgram/ml of G418 (GibcoBRL) for 15 days.

The DNA construct can be stably introduced into a donor somatic cell line by lipofection using a protocol such as the following: about 2 x 10⁵ cells are plated into a 3.5 cmiameter well and transfected with 2 micrograms of linearized DNA using LipfectAMINE™ (GibcoBRL). Forty-eight hours after transfection, the cells are split 1:1000 and 1:5000 and, if the DNA construct contains a neomyosin resistance gene for selection, G418 is added to a final concentration of 0.35 mg/ml. Neomyocin resistant clones are isolated and expanded for cyropreservation as well as nuclear transfer.

### Tissue-Specific Expression of Proteins

It is often desirable to express a heterologous protein, e.g., a mutated form of ATIII, in a specific tissue or fluid, e.g., the milk, of a transgenic animal. The heterologous protein can be recovered from the tissue or fluid in which it is expressed. For example, it is often desirable to express the heterologous protein in milk. Methods for producing a heterologous protein under the control of a milk specific promoter are described below. In addition, other tissue-specific promoters, as well as, other regulatory elements, e.g., signal sequences and sequence which enhance secretion of non-secreted proteins, are described below.

### Milk Specific Promoters

Useful transcriptional promoters are those promoters that are preferentially activated in mammary epithelial cells, including promoters that control the genes encoding milk proteins such as caseins, beta lactoglobulin (Clark et al., (1989) Bio/Technology 7: 487-492), whey acid protein (Gordon et al. (1987) Bio/Technology 5: 1183-1187), and lactalbumin (Soulier et al., (1992) FEBS Letts. 297: 13). Casein promoters may be derived from the alpha, beta, gamma or kappa casein genes of any mammalian species; a preferred promoter is derived from the goat beta casein gene (DiTullio, (1992) Bio/Technology 10:74-77). Milk-specific protein promoter or the promoters that are specifically activated in mammary tissue can be derived from cDNA or genomic sequences. Preferably, they are genomic in origin.

DNA sequence information is available for the mammary gland specific genes listed above, in at least one, and often in several organisms. See, e.g., Richards et al., J. Biol. Chem. 256, 526-532 (1981) (α-lactalbumin rat); Campbell et al., Nucleic Acids Res. 12, 8685-8697 (1984) (rat WAP); Jones et al., J. Biol. Chem. 260, 7042-7050 (1985) (rat β-casein); Yu-Lee & Rosen, J. Biol. Chem. 258, 10794-10804 (1983) (rat γ-casein); Hall, Biochem. J. 242, 735-742 (1987) (α-lactalbumin human); Stewart, Nucleic Acids Res. 12, 389 (1984) (bovine αs1 and κ casein cDNAs); Gorodetsky et al., Gene 66, 87-96 (1988) (bovine β casein); Alexander et al., Eur. J. Biochem. 178, 395-401 (1988) (bovine κ casein); Brignon et al., FEBS Lett. 188, 48-55 (1977) (bovine αS2 casein); Jamieson et al., Gene 61, 85-90 (1987), Ivanov et al., Biol. Chem. Hoppe-Seyler 369, 425-429 (1988), Alexander et al., Nucleic Acids Res. 17, 6739 (1989) (bovine 13 lactoglobulin); Vilotte et al., Biochimie 69, 609-620 (1987) (bovine α-lactalbumin). The structure and function of the various milk protein genes are reviewed by Mercier & Vilotte, J. Dairy Sci. 76, 3079-3098 (1993) (incorporated by reference in its entirety for all purposes). If additional flanking sequence are useful in optimizing expression of the heterologous protein, such sequences can be cloned using the existing sequences as probes. Mammary-gland specific regulatory sequences from different organisms can be obtained by screening libraries from such organisms using known cognate nucleotide sequences, or antibodies to cognate proteins as probes.

### Signal Sequences

Useful signal sequences are milk-specific signal sequences or other signal sequences which result in the secretion of eukaryotic or prokaryotic proteins. Preferably, the signal sequence is selected from milk-specific signal sequences, i.e., it is from a gene which encodes a product secreted into milk. Most preferably, the milk-specific signal sequence is related to the milk-specific promoter used in the construct, which are described below. The size of the signal sequence is not critical. All that is required is that the sequence be of a sufficient size to effect secretion of the desired recombinant protein, e.g., in the mammary tissue. For example, signal sequences from genes coding for caseins, e.g., alpha, beta, gamma or kappa caseins, beta lactoglobulin, whey acid protein, and lactalbumin can be used. A preferred signal sequence is the goat β-casein signal sequence.

Signal sequences from other secreted proteins, e.g., proteins secreted by kidney cells, pancreatic cells or liver cells, can also be used. Preferably, the signal sequence results in the secretion of proteins into, for example, urine or blood.

### Amino-Terminal Regions of Secreted Proteins

A non-secreted protein can also be modified in such a manner that it is secreted such as by inclusion in the protein to be secreted of all or part of the coding sequence of a protein which is normally secreted. Preferably the entire sequence of the protein which is normally secreted is not included in the sequence of the protein but rather only a sufficient portion of the amino terminal end of the protein which is normally secreted to result in secretion of the protein. For example, a protein which is not normally secreted is fused (usually at its amino terminal end) to an amino terminal portion of a protein which is normally secreted.

In one aspect, the protein which is normally secreted is a protein which is normally secreted in milk. Such proteins include proteins secreted by mammary epithelial cells, milk proteins such as caseins, beta lactoglobulin, whey acid protein , and lactalbumin. Casein proteins include alpha, beta, gamma or kappa casein genes of any mammalian species. A preferred protein is beta casein, e.g., goat beta casein. The sequences which encode the secreted protein can be derived from either cDNA or genomic sequences. Preferably, they are genomic in origin, and include one or more introns.

### Other Tissue-Specific Promoters

Other tissue-specific promoters which provide expression in a particular tissue can be used. Tissue specific promoters are promoters which are expressed more strongly in a particular tissue than in others. Tissue specific promoters are often expressed essentially exclusively in the specific tissue.

Tissue-specific promoters which can be used include: a neural-specific promoter, e.g., nestin, Wnt-1, Pax-1, Engrailed-1, Engrailed-2, Sonic hedgehog; a liver-specific promoter, e.g., albumin, alpha-1 antirypsin; a muscle-specific promoter, e.g., myogenin, actin, MyoD, myosin; an oocyte specific promoter, e.g., ZP1, ZP2, ZP3; a testes-specific promoter, e.g., protamin, fertilin, synaptonemal complex protein-1; a blood-specific promoter, e.g., globulin, GATA-1, porphobilinogen deaminase; a lung-specific promoter, e.g., surfactant protein C; a skin- or wool-specific promoter, e.g., keratin, elastin; endothelium-specific promoters, e.g., Tie-1, Tie-2; and a bone-specific promoter, e.g., BMP.

In addition, general promoters can be used for expression in several tissues. Examples of general promoters include β-actin, ROSA-21, PGK, FOS, c-myc, Jun-A, and Jun-B.

### Insulator Sequences

The DNA constructs used to make a transgenic animal can include at least one insulator sequence. The terms "insulator", "insulator sequence" and "insulator element" are used interchangeably herein. An insulator element is a control element which insulates the transcription of genes placed within its range of action but which does not perturb gene expression, either negatively or positively. Preferably, an insulator sequence is inserted on either side of the DNA sequence to be transcribed. For example, the insulator can be positioned about 200 bp to about 1 kb, 5' from the promoter, and at least about 1 kb to 5 kb from the promoter, at the 3' end of the gene of interest. The distance of the insulator sequence from the promoter and the 3' end of the gene of interest can be determined by those skilled in the art, depending on the relative sizes of the gene of interest, the promoter and the enhancer used in the construct. In addition, more than one insulator sequence can be positioned 5' from the promoter or at the 3' end of the transgene. For example, two or more insulator sequences can be positioned 5' from the promoter. The insulator or insulators at the 3' end of the transgene can be positioned at the 3' end of the gene of interest, or at the 3'end of a 3' regulatory sequence, e.g., a 3' untranslated region (UTR) or a 3' flanking sequence.

A preferred insulator is a DNA segment which encompasses the 5' end of the chicken β-globin locus and corresponds to the chicken 5' constitutive hypersensitive site as described in PCT Publication 94/23046, the contents of which is incorporated herein by reference.

### DNA Constructs

A cassette which encodes a heterologous protein can be assembled as a construct which includes a promoter for a specific tissue, e.g., for mammary epithelial cells, e.g., a casein promoter, e.g., a goat beta casein promoter, a milk-specific signal sequence, e.g., a casein signal sequence, e.g., a β-casein signal sequence, and a DNA encoding the heterologous protein.

The construct can also include a 3' untranslated region downstream of the DNA sequence coding for the non-secreted protein. Such regions can stabilize the RNA transcript of the expression system and thus increases the yield of desired protein from the expression system. Among the 3' untranslated regions useful in the constructs for use in the invention are sequences that provide a poly A signal. Such sequences may be derived, e.g., from the SV40 small t antigen, the casein 3' untranslated region or other 3' untranslated sequences well known in the art. In one aspect, the 3' untranslated region is derived from a milk specific protein. The length of the 3' untranslated region is not critical but the stabilizing effect of its poly A transcript appears important in stabilizing the RNA of the expression sequence.

Optionally, the construct can include a 5' untranslated region between the promoter and the DNA sequence encoding the signal sequence. Such untranslated regions can be from the same control region from which promoter is taken or can be from a different gene, e.g., they may be derived from other synthetic, semi-synthetic or natural sources. Again their specific length is not critical, however, they appear to be useful in improving the level of expression.

The construct can also include about 10%, 20%, 30%, or more of the N-terminal coding region of a gene preferentially expressed in mammary epithelial cells. For example, the N-terminal coding region can correspond to the promoter used, e.g., a goat β-casein N-terminal coding region.

The construct can be prepared using methods known in the art. The construct can be prepared as part of a larger plasmid. Such preparation allows the cloning and selection of the correct constructions in an efficient manner. The construct can be located between convenient restriction sites on the plasmid so that they can be easily isolated from the remaining plasmid sequences for incorporation into the desired mammal.

### Oocytes

Oocytes for use in the invention can be obtained at various times during an animal's reproductive cycle. Oocytes at various stages of the cell cycle can be obtained and then induced *in vitro* to enter a particular stage of meiosis. For example, oocytes cultured on serum-starved medium become arrested in metaphase. In addition, arrested oocytes can be induced to enter telophase by serum activation.

Oocytes can be matured *in vitro* before they are used to form a reconstructed embryo. This process usually requires collecting immature oocytes from mammalian ovaries, e.g., a caprine ovary, and maturing the oocyte in a medium prior to enucleation until the oocyte reaches the desired meiotic stage, e.g., metaphase or telophase. In addition, oocytes that have been matured *in vivo* can be used to form a reconstructed embryo.

Oocytes can be collected from a female mammal during superovulation. Briefly, oocytes, e.g., caprine oocytes, can be recovered surgically by flushing the oocytes from the oviduct of the female donor. Methods of inducing superovulation in goats and the collection of caprine oocytes is described herein.

### Transfer of Reconstructed Embryos

A reconstructed embryo can be transferred to a recipient doe and allowed to develop into a cloned or transgenic mammal. For example, the reconstructed embryo can be transferred via the fimbria into the oviductal lumen of each recipient doe. In addition, methods of transferring an embryo to a recipient mammal are known in the art and described, for example, in Ebert et al. (1994) Bio/Technology 12:699.

### Purification of Proteins from Milk

The transgenic protein, e.g., a mutated form of ATIII, can be produced in milk at relatively high concentrations and in large volumes, providing continuous high level output of normally processed peptide that is easily harvested from a renewable resource. There are several different methods known in the art for isolation of proteins form milk.

Milk proteins usually are isolated by a combination of processes. Raw milk first is fractionated to remove fats, for example, by skimming, centrifugation, sedimentation (H.E. Swaisgood, Developments in Dairy Chemistry, I: Chemistry of Milk Protein, Applied Science Publishers, NY, 1982), acid precipitation (U.S. Patent No. 4,644,056) or enzymatic coagulation with rennin or chymotrypsin (Swaisgood, ibid.). Next, the major milk proteins may be fractionated into either a clear solution or a bulk precipitate from which the specific protein of interest may be readily purified.

French Patent No. 2487642 describes the isolation of milk proteins from skim milk or whey by membrane ultrafiltration in combination with exclusion chromatography or ion exchange chromatography. Whey is first produced by removing the casein by coagulation with rennet or lactic acid. U.S. Patent No. 4,485,040 describes the isolation of an alpha-lactoglobulin-enriched product in the retentate from whey by two sequential ultrafiltration steps. U.S. Patent No. 4,644,056 provides a method for purifying immunoglobulin from milk or colostrum by acid precipitation at pH 4.0-5.5, and sequential cross-flow filtration first on a membrane with 0.1 - 1.2 micrometer pore size to clarify the product pool and then on a membrane with a separation limit of 5 - 80 kd to concentrate it.

Similarly, U.S. Patent No. 4,897,465 teaches the concentration and enrichment of a protein such as immunoglobulin from blood serum, egg yolks or whey by sequential ultrafiltration on metallic oxide membranes with a pH shift. Filtration is carried out first at a pH below the isoelectric point (pI) of the selected protein to remove bulk contaminants from the protein retentate, and next at a pH above the pI of the selected protein to retain impurities and pass the selected protein to the permeate. A different filtration concentration method is taught by European Patent No. EP 467 482 B1 in which defatted skim milk is reduced to pH 3-4, below the pI of the milk proteins, to solubilize both casein and whey proteins. Three successive rounds of ultrafiltration or diafiltration then concentrate the proteins to form a retentate containing 15-20% solids of which 90% is protein. Alternatively, British Patent Application No. 2179947 discloses the isolation of lactoferrin from whey by ultrafiltration to concentrate the sample, followed by weak cation exchange chromatography at approximately a neutral pH. No measure of purity is reported. In PCT Publication No. WO 95/22258, a protein such as -lactoferrin is recovered from milk that has been adjusted to high ionic strength by the addition of concentrated salt, followed by cation exchange chromatography.

In all of these methods, milk or a fraction thereof is first treated to remove fats, lipids, and other particulate matter that would foul filtration membranes or chromatography media. The initial fractions thus produced may consist of casein, whey, or total milk protein, from which the protein of interest is then isolated.

PCT Patent Publication No. WO 94/19935 discloses a method of isolating a biologically active protein from whole milk by stabilizing the solubility of total milk proteins with a positively charged agent such as arginine, imidazole or Bis-Tris. This treatment forms a clarified solution from which the protein may be isolated, e.g., by filtration through membranes that otherwise would become clogged by precipitated proteins.

USSN 08/648,235 discloses a method for isolating a soluble milk component, such as a peptide, in its biologically active form from whole milk or a milk fraction by tangential flow filtration. Unlike previous isolation methods, this eliminates the need for a first fractionation of whole milk to remove fat and casein micelles, thereby simplifying the process and avoiding losses of recovery and bioactivity. This method may be used in combination with additional purification steps to further remove contaminants and purify the product, e.g., protein, of interest.

### Production of Fragments and Analogs

One skilled in the art can alter the disclosed structure of ATIII by producing fragments or analogs, and test the newly produced structures for activity. Examples of prior art methods which allow the production and testing of fragments and analogs are discussed below. These, or other methods, can be used to make and screen fragments and analogs of a ATIII polypeptide. In preferred embodiments, the ATIII structure modified is human ATIII.

### Generation of Fragments

Fragments of a protein can be produced in several ways, e.g., recombinantly, by proteolytic digestion, or by chemical synthesis. Internal or terminal fragments of a polypeptide can be generated by removing one or more nucleotides from one end (for a terminal fragment) or both ends (for an internal fragment) of a nucleic acid which encodes the polypeptide. Expression of the mutagenized DNA produces polypeptide fragments. Digestion with "end-nibbling" endonucleases can thus generate DNA's which encode an array of fragments. DNA's which encode fragments of a protein can also be generated by random shearing, restriction digestion or a combination of the above-discussed methods.

Fragments can also be chemically synthesized using techniques known in the art such as conventional Merrifield solid phase f-Moc or t-Boc chemistry. For example, peptides of the present invention may be arbitrarily divided into fragments of desired length with no overlap of the fragments, or divided into overlapping fragments of a desired length.

### Generation of Analogs: Production of Altered DNA and Peptide Sequences by Random Methods

Amino acid sequence variants of a protein can be prepared by random mutagenesis of DNA which encodes a protein or a particular domain or region of a protein. Useful methods include PCR mutagenesis and saturation mutagenesis. A library of random amino acid sequence variants can also be generated by the synthesis of a set of degenerate oligonucleotide sequences. (Methods for screening proteins in a library of variants are elsewhere herein.)

### PCR Mutagenesis

In PCR mutagenesis, reduced Taq polymerase fidelity is used to introduce random mutations into a cloned fragment of DNA (Leung et al., 1989, Technique 1:11-15). This is a very powerful and relatively rapid method of introducing random mutations. The DNA region to be mutagenized is amplified using the polymerase chain reaction (PCR) under conditions that reduce the fidelity of DNA synthesis by Taq DNA polymerase, e.g., by using a dGTP/dATP ratio of five and adding Mn²⁺ to the PCR reaction. The pool of amplified DNA fragments are inserted into appropriate cloning vectors to provide random mutant libraries.

### Saturation Mutagenesis

Saturation mutagenesis allows for the rapid introduction of a large number of single base substitutions into cloned DNA fragments (Mayers et al., 1985, Science 229:242). This technique includes generation of mutations, e.g., by chemical treatment or irradiation of single-stranded DNA *in vitro,* and synthesis of a complimentary DNA strand. The mutation frequency can be modulated by modulating the severity of the treatment, and essentially all possible base substitutions can be obtained. Because this procedure does not involve a genetic selection for mutant fragments both neutral substitutions, as well as those that alter function, are obtained. The distribution of point mutations is not biased toward conserved sequence elements.

### Degenerate Oligonucleotides

A library of homologs can also be generated from a set of degenerate oligonucleotide sequences. Chemical synthesis of a degenerate sequences can be carried out in an automatic DNA synthesizer, and the synthetic genes then ligated into an appropriate expression vector. The synthesis of degenerate oligonucleotides is known in the art (see for example, Narang, SA (1983) Tetrahedron 39:3; Itakura et al. (1981) Recombinant DNA. Proc 3rd Cleveland Sympos. Macromolecules, ed. AG Walton, Amsterdam: Elsevier pp273-289; Itakura et al. (1984) Annu. Rev. Biochem. 53:323; Itakura et al. (1984) Science 198:1056; Ike et al. (1983) Nucleic Acid Res. 11:477. Such techniques have been employed in the directed evolution of other proteins (see, for example, Scott et al. (1990) Science 249:386-390; Roberts et al. (1992) PNAS 89:2429-2433; Devlin et al. (1990) Science 249: 404-406; Cwirla et al. (1990) PNAS 87: 6378-6382; as well as U.S. Patents Nos. 5,223,409, 5,198,346, and 5,096,815).

### Generation of Analogs: Production of Altered DNA and Peptide Sequences by Directed Mutagenesis

Non-random or directed, mutagenesis techniques can be used to provide specific sequences or mutations in specific regions. These techniques can be used to create variants which include, e.g., deletions, insertions, or substitutions, of residues of the known amino acid sequence of a protein. The sites for mutation can be modified individually or in series, e.g., by (1) substituting first with conserved amino acids and then with more radical choices depending upon results achieved, (2) deleting the target residue, or (3) inserting residues of the same or a different class adjacent to the located site, or combinations of options 1-3.

### Alanine Scanning Mutagenesis

Alanine scanning mutagenesis is a useful method for identification of certain residues or regions of the desired protein that are preferred locations or domains for mutagenesis, Cunningham and Wells (Science 244:1081-1085, 1989). In alanine scanning, a residue or group of target residues are identified (e.g., charged residues such as Arg, Asp, His, Lys, and Glu) and replaced by a neutral or negatively charged amino acid (most preferably alanine or polyalanine). Replacement of an amino acid can affect the interaction of the amino acids with the surrounding aqueous environment in or outside the cell. Those domains demonstrating functional sensitivity to the substitutions are then refined by introducing further or other variants at or for the sites of substitution. Thus, while the site for introducing an amino acid sequence variation is predetermined, the nature of the mutation per se need not be predetermined. For example, to optimize the performance of a mutation at a given site, alanine scanning or random mutagenesis may be conducted at the target codon or region and the expressed desired protein subunit variants are screened for the optimal combination of desired activity.

### Oligonucleotide-Mediated Mutagenesis

Oligonucleotide-mediated mutagenesis is a useful method for preparing substitution, deletion, and insertion variants of DNA, see, e.g., Adelman et al., (DNA 2:183, 1983). Briefly, the desired DNA is altered by hybridizing an oligonucleotide encoding a mutation to a DNA template, where the template is the single-stranded form of a plasmid or bacteriophage containing the unaltered or native DNA sequence of the desired protein. After hybridization, a DNA polymerase is used to synthesize an entire second complementary strand of the template that will thus incorporate the oligonucleotide primer, and will code for the selected alteration in the desired protein DNA. Generally, oligonucleotides of at least 25 nucleotides in length are used. An optimal oligonucleotide will have 12 to 15 nucleotides that are completely complementary to the template on either side of the nucleotide(s) coding for the mutation. This ensures that the oligonucleotide will hybridize properly to the single-stranded DNA template molecule. The oligonucleotides are readily synthesized using techniques known in the art such as that described by Crea et al. (Proc. Natl. Acad. Sci. USA, 75: 5765[1978]).

### Cassette Mutagenesis

Another method for preparing variants, cassette mutagenesis, is based on the technique described by Wells et al. (Gene, 34:315[1985]). The starting material is a plasmid (or other vector) which includes the protein subunit DNA to be mutated. The codon(s) in the protein subunit DNA to be mutated are identified. There must be a unique restriction endonuclease site on each side of the identified mutation site(s). If no such restriction sites exist, they may be generated using the above-described oligonucleotide-mediated mutagenesis method to introduce them at appropriate locations in the desired protein subunit DNA. After the restriction sites have been introduced into the plasmid, the plasmid is cut at these sites to linearize it. A double-stranded oligonucleotide encoding the sequence of the DNA between the restriction sites but containing the desired mutation(s) is synthesized using standard procedures. The two strands are synthesized separately and then hybridized together using standard techniques. This double-stranded oligonucleotide is referred to as the cassette. This cassette is designed to have 3' and 5' ends that are comparable with the ends of the linearized plasmid, such that it can be directly ligated to the plasmid. This plasmid now contains the mutated desired protein subunit DNA sequence.

### Combinatorial Mutagenesis

Combinatorial mutagenesis can also be used to generate mutants. E.g., the amino acid sequences for a group of homologs or other related proteins are aligned, preferably to promote the highest homology possible. All of the amino acids which appear at a given position of the aligned sequences can be selected to create a degenerate set of combinatorial sequences. The variegated library of variants is generated by combinatorial mutagenesis at the nucleic acid level, and is encoded by a variegated gene library. For example, a mixture of synthetic oligonucleotides can be enzymatically ligated into gene sequences such that the degenerate set of potential sequences are expressible as individual peptides, or alternatively, as a set of larger fusion proteins containing the set of degenerate sequences.

### Examples

All mutants were produced in a pUC vector by gap mutagenesis in the case of T85M, T85K, N187D, N135Q and K114Q. Oligonucleotides were synthesized (Operon) with changes at the desired codon to change its amino acid designation and also silent mutations were introduced at neighboring codons to exaggerate the difference between the wild type and the mutant in hybridization screening. The following oligonucleotides were used: 
T85M:CCTGAGTATCagtAtGGCcTTTGCTATGAS
L S I S M A F A M
T85K:CCTGAGTATCAGTAAGGCCTTTGCTATGAC
L S I S K A F A M
N187D:CAGAGCGGCCATagACAAgTGGGTGTCC
R A A I D K W V S
N135Q:CTATCGAAAAGCacagAAgTCCTCCAAGTTAG
Y R K A Q K S S K L
 
K114Q:CACCATATCgGAacAAACcTCTGATCAGA
T I S E Q T S D Q
 The gap mutagenesis was preformed as previously described. Briefly, the plasmid pAT-7 was digested at the unique restriction sites SacII and HpaI and the vector was gel purified from the 600 bp insert and extracted from the agarose using Gene Clean (Bio101). This fragment was combined with a similar quantity of XmnI digested pAT-7 and the phosphorylated mutant oligo then boiled in a water bath for 7 minutes. The fragments were then allowed to reanneal at 37C for 30 minutes. The gap was filled in and ligated with Klenow and T4 Ligase in 1xligation buffer. After transformation into XL-1 Blue supercompetent cells and plating on nitrocellulose filters on Lbamp plates, the filters were replicated and screened by hybridization utilizing the kinased mutant oligo. The temperature of the hybridization was set at 5C below the melting temperature of the oligo to differentiate between the mutant and the wild type.

Two truncated mutants were created using either PCR in the case of the truncation at the elastase site and an oligo in the case of the thrombin site. The elastase truncated mutant was PCR'ed using an Oligo which was 5' of the Xho1 site in pAT-7 and an oligo that was homologous to the sequence 5' of the elastase site with a stop codon and a Sall adaptor sequence. PCR was performed using Pfu polymerase and the manufacturer's buffer with the reannealing temperature 4C below the calculated melting temperature. The PCR product was gel purified and after digestion with Sal1 and Xho1, ligated into a modified pUC19 vector. The ATIII truncated at the thrombin site was created by inserting a double stranded oligonucleotide (Operon, see below) with Nsi1 and Xho1 ends and a Bg12 site and a stop codon at the 3' end, into the wild type cDNA clone.

The deglycosylated form was synthesized as a Sac2-Hpa1 restriction fragment changing all four glycosylation site asparagines into glutamines and optimizing the codon usage for mammalian expression and eliminating a destabilization sequence by Operon and ligated into the Sac2-Hpa1 restricted wild type. All mutants except the thrombin truncated and N 135Q were subcloned into a mammalian expression vector pCEP-4 (Invitrogen) and transfected into a strain of 293 cells which are positive for the EBNA-1 nuclear protein using LipofectAmine Plus (Life Technologies). This system typically gives an 80-90% transfection efficiency based on beta galactose staining. After 48 hours conditioned media and cell lysates were run on a 10-18% SDS-PAGE and western blotted. The blot was probed with an HRP conjugated anti-human (ATIII (Biodesign International).

Transgenic vectors (BC800 and BC350) containing the goat beta casein promoter, 3' UTR and insulator sequences were used to subclone these mutant inserts for microinjection into mouse oocytes. Five mutants have been injected so far, thrombin site truncated (BC896), elastase site truncated (BC898), non-glycosylated (BC897), beta form (BC899) and thrombin truncated non-glycosylated (BC929). Four have had founders identified using an ATIII specific primer set on tail DNA preps (standard proteinase K protocol). A low percentage of transgenics were produced about 5-8% and these were mated to generate milk and F1 progeny. Milk from the thrombin site truncated ATIII was analyzed by western blot and one mouse was producing in excess of 0.5 mg/mL and another mouse in excess of 2 mg/mL. Although much of this is aggregated there is still a substantial portion which is migrating at the level of the monomer. In addition to this evidence that the protein is properly folded the milk was also looked at in an ELISA format where a constant amount of ATIII, either wild type or truncated was captured by a goat antihuman ATIII polyclonal antibodies and then a peroxidase conjugated sheep antihuman ATIII was titrated against that. Titration curves were very close suggesting that most of the epitopes are available and it must be properly folded. Milk of the non-glycosylated mutant was also analyzed by western blot. Even under reducing conditions most of the material remained aggregated. Nonreduced lane was almost entirely aggregated. Milk of beta ATIII founder females secreted low amounts of protein into the milk.

Most of the constructs were tested for expression in tissue culture but due to the nature of generating transgenic mice only selected forms were micro-injected. The results of these experiments have been summarized in Table II. In all tissue culture experiments large amounts of the proteins were expressed in the cells but in only two examples, the wild type ATIII and the conservative mutant wibble (T85M) did significant amounts of the proteins get out of the cells. The majority of the material in the cells appears to not be processed into the mature form. Five ATIII variants have been made in transgenic mice so far. Two truncated forms one at the thrombin site (GTC896) and the other at the elastase cleavage site (GTC898), the thrombin cleavage site truncated form non-glycosylated (GTC928), a non-glycosylated form itself (GTC897) and the feta form N135Q (GTC899). Founders were identified by PCR analysis of mouse tail DNA and bred to generate F1 progeny and milk for secretion analysis. All five variants have been found to be secreted into the milk of these lactating mice by western analysis. Under non-reducing conditions all of the forms appear to have a high amount of aggregation especially the non-glycosylated form.

**Table 1. List of Constructs**

| **ATIII Mutants** | **pUC** | **pCEP4** | **pBC** |
|---|---|---|---|
| Wild Type ATIII | pAT7 | 845 | 197 |
| T85M Wibble | 843 | 852 | 855 |
| T85K Wobble | 844 | 853 | 856 |
| N187D Rouen VI | 849 | 858 | |
| N135Q Beta Form | 872 | | 899 |
| K114Q Non-heparin Binding | 880 | 888 | |
| Deglycosylated | 879 | 887 | 897 |
| Truncated a) Elastase Site | 881 | 889 | 898 |
| Truncated b) Thrombin Site | 884 | 920 | 896 |
| Truncated/Non-glycosylated | 916 | 928 | 929 |
| Truncated (Thromb. Site): hSA | 925 | 932 | 933 |
| Fusion | | | |
| Beta Casein:PF4/47-70 Fusion | 927 | | 931 |
| PF4/47-70 | 935 | | |
| hSA:PF4/47-70 Fusion | 936 | 956 | 943 |

**Table 2. Expression of ATIII and tgATIIIₘᵤₜ Constructs**

| **ATIII Mutants** | **Tissue Culture** | **Milk** | **Heparin Binding** |
|---|---|---|---|
| Wild Type | 1 | 2000 | + |
| T85M Wibble | 0.5 | | |
| T85K Wobble | - | | |
| N187Q Rouen VI | - | | |
| N135Q Beta Form | | 500 | |
| K114Q Non-heparin Binding | - | | |
| Non-glycosylated | - | 1000 | |
| Truncated a) Elastase Site | - | 1000 | + |
| Truncated b) Thrombin Site | - | 2000 | |
| Truncated (Thromb. Site)/ | - | 500 | |
| Non-glycosylated | | | |
| Truncated (Thromb. Site): | | | |
| hSA | | | |

## Claims

1. A transgenically produced mutated human Antithrombin III (tgATIII ₘᵤₜ) having an antiangiogenic activity, wherein the reactive loop of ATIII is unreactive for use as a medicament.

2. The mutated ATIII of claim 1, wherein the reactive loop is folded.

3. The mutated ATIII of claim 1, wherein the reactive loop is partially or wholly removed.

4. The mutated ATIII of claim 2, wherein the ATIII is mutated by altering at least one alpha helix underlying the beta sheet.

5. The mutated ATIII of claim 4, wherein the alpha helix is altered by insertion, deletion, or a substitution of at least one nucleotide of the sequence encoding an alpha helix of ATIII.

6. The mutated ATIII of claim 5, wherein a threonine at position 85 of human ATIII is changed to a methionine.

7. The mutated ATIII of claim 5, wherein a threonine at position 85 of human ATIII is changed to lysine.

8. The mutated ATIII of claim 5, wherein an asparagine at position 187 of human ATIII is changed to an aspartic acid.

9. The mutated ATIII of claim 3, wherein ATIII is mutated by removing a portion or all of the C-terminus of ATIII.

10. The mutated ATIII of claim 9, wherein about 50 amino acids or less are removed from the C-terminus.

11. The mutated ATIII of claim 10, wherein the C-terminus is removed at an elastase site of ATIII.

12. The mutated ATIII of claim 10, wherein the C-terminus is removed at a thrombin cleavage site of ATIII.

13. The mutated ATIII of claim 10, wherein the reactive loop of ATIII is altered by allowing the beta strand of the reactive loop to fold into the beta sheet.

14. A method of making transgenic tgATIII ₘᵤₜ comprising:
providing a transgenic non-human mammal which includes a DNA sequence encoding a tgATIII ₘᵤₜ having an antiangiogenic activity, wherein the reactive loop of ATIII is unreactive, under control of a milk specific promoter;
allowing the tgATIII ₘᵤₜ to be expressed, thereby making tgATIII ₘᵤₜ.

15. The method of claim 14, wherein the mammal is selected from the group consisting of a goat, a sheep, a cow, a pig, a rabbit, a rat and a mouse.

16. The method of claim 14, wherein the milk specific promoter is selected from the group consisting of: a casein promoter, a beta lactoglobulin promoter, a whey acid protein promoter, and a lactalbumin promoter.

## Patentansprüche

1. Transgen hergestelltes mutiertes humanes Antithrombin III (tgATIIIₘᵤₜ) mit einer antiangiogenen Aktivität, wobei die reaktive Schleife von ATIII reaktionsunfähig ist, zur Verwendung als Medikament.

2. Mutiertes ATIII nach Anspruch 1, wobei die reaktive Schleife gefaltet ist.

3. Mutiertes ATIII nach Anspruch 1, wobei die reaktive Schleife teilweise oder vollständig entfernt ist.

4. Mutiertes ATIII nach Anspruch 2, wobei das ATIII durch Ändern von mindestens einer alpha-Helix, zugrundeliegend dem beta-Blatt, mutiert ist.

5. Mutiertes ATIII nach Anspruch 4, wobei die alpha-Helix durch Insertion, Deletion oder eine Substitution von mindestens einem Nucleotid der Sequenz, codierend eine alpha-Helix von ATIII, geändert ist.

6. Mutiertes ATIII nach Anspruch 5, wobei ein Threonin an Position 85 von humanem ATIII zu einem Methionin verändert ist.

7. Mutiertes ATIII nach Anspruch 5, wobei ein Threonin an Position 85 von humanem ATIII zu Lysin verändert ist.

8. Mutiertes ATIII nach Anspruch 5, wobei ein Asparagin an Position 187 von humanem ATIII zu einer Asparaginsäure verändert ist.

9. Mutiertes ATIII nach Anspruch 3, wobei ATIII durch Entfernen eines Teils oder des gesamten C-Terminus von ATIII mutiert ist.

10. Mutiertes ATIII nach Anspruch 9, wobei etwa 50 Aminosäuren oder weniger von dem C-Terminus entfernt sind.

11. Mutiertes ATIII nach Anspruch 10, wobei der C-Terminus an einer Elastasestelle von ATIII entfernt ist.

12. Mutiertes ATIII nach Anspruch 10, wobei der C-Terminus an einer Thrombinspaltstelle von ATIII entfernt ist.

13. Mutiertes ATIII nach Anspruch 10, wobei die reaktive Schleife von ATIII geändert ist, indem dem beta-Strang der reaktiven Schleife erlaubt ist, in das beta-Blatt zu falten.

14. Verfahren zum Herstellen von transgenem tgATIIIₘᵤₜ, umfassend:
Bereitstellen eines transgenen nichthumanen Säugers, welcher eine DNA-Sequenz, codierend ein tgATIIIₘᵤₜ mit einer antiangiogenen Aktivität, einschließt, wobei die reaktive Schleife von ATIII reaktionsunfähig ist, unter Kontrolle eines milchspezifischen Promotors,
Erlauben, daß das tgATIIIₘᵤₜ exprimiert wird, **dadurch** Herstellen von tgATIIIₘᵤₜ.

15. Verfahren nach Anspruch 14, wobei der Säuger aus der Gruppe, bestehend aus einer Ziege, einem Schaf, einer Kuh, einem Schwein, einem Kaninchen, einer Ratte und einer Maus, ausgewählt ist.

16. Verfahren nach Anspruch 14, wobei der milchspezifische Promotor aus der Gruppe bestehend aus:
einem Casein-Promotor, einem beta-Lactoglobulin-Promotor, einem Molke-Säure-Protein-Promotor und einen Lactalbumin-Promotor, ausgewählt ist.

## Revendications

1. Antithrombine III (tgATIIImut) humaine mutée produite par voie transgénique ayant une activité anti-angiogénique, dans laquelle la boucle réactive de l'ATIII est non réactive, à utiliser en tant que médicament.

2. L'ATIII mutée de la revendication 1, dans laquelle la boucle réactive est pliée.

3. L'ATIII mutée de la revendication 1, dans laquelle la boucle réactive est partiellement ou complètement retirée.

4. L'ATIII mutée de la revendication 2, dans laquelle l'ATIII est mutée en modifiant au moins une hélice alpha sous-jacente au feuillet bêta.

5. L'ATIII mutée de la revendication 4, dans laquelle l'hélice alpha est modifiée par l'insertion, la délétion ou la substitution d'au moins un nucléotide de la séquence codant une hélice alpha d'ATIII.

6. L'ATIII mutée de la revendication 5, dans laquelle une thréonine à la position 85 de l'ATIII humaine est changée en méthionine.

7. L'ATIII mutée de la revendication 5, dans laquelle une thréonine à la position 85 de l'ATIII humaine est changée en lysine.

8. L'ATIII mutée de la revendication 5, dans laquelle une aspargine à la position 187 de l'ATIII humaine est changée en un acide aspartique.

9. L'ATIII mutée de la revendication 3, dans laquelle l'ATIII est mutée en retirant une partie ou toute la terminaison C d'ATIII.

10. L'ATIII mutée de la revendication 9, dans laquelle environ 50 acides aminés ou moins sont retirés de la terminaison C.

11. L'ATIII mutée de la revendication 10, dans laquelle la terminaison C est retirée à un site élastase d'ATIII.

12. L'ATIII mutée de la revendication 10, dans laquelle la terminaison C est retirée à un site de clivage de la thrombine d'ATIII.

13. L'ATIII mutée de la revendication 10, dans laquelle la boucle réactive d'ATIII est modifiée en permettant au brin bêta de la boucle réactive de se plier dans le feuillet bêta.

14. Procédé de préparation de tgATIIIm transgénique, comprenant:
fournir un mammifère transgénique non humain qui comprend une séquence d'ADN codant une tgATIIImut ayant une activité anti-angiogénique, où la boucle réactive d'ATIII est non réactive, sous le contrôle d'un promoteur spécifique du lait;
permettre que tgATIIImut soit exprimée, faisant ainsi tgATIIImut.

15. Le procédé de la revendication 14, dans lequel le mammifère est sélectionné parmi le groupe constitué d'une chèvre, d'un mouton, d'une vache, d'un cochon, d'un lapin, d'un rat et d'une souris.

16. Le procédé de la revendication 14, dans lequel le promoteur spécifique du lait est sélectionné parmi le groupe consistant en: un promoteur de caséine, un promoteur de β-lactoglobuline, un promoteur de protéine acide du lactosérum et un promoteur de lactalbumine.
